# EUROPEAN PATENT SPECIFICATION

(11) **EP 1 925 273 B1**
(45) Date of publication and mention of the grant of the patent: **29.07.2009**
(21) Application number: 07022551.1
(22) Date of filing: 21.11.2007
(51) Int. Cl.: A61F 7/10

(54) **Device for providing instant cooling, particularly for treating parts of the human body or items in general**
Vorrichtung zur sofortigen Kühlung, insbesondere zur Behandlung menschlicher Körperteile oder sonstiger allgemeiner Elemente
Dispositif de fourniture de refroidissement instantané, particulièrement de traitement de parties du corps humain ou d'éléments en général

(30) Priority: 27.11.2006 IT MI20062278
(43) Date of publication of application: 28.05.2008
(73) Proprietor: Ferrari Trading S.r.l., 24069 Trescore Balneario (BG) (IT)
(72) Inventor: Ferri, Carlo, 24022 Alzano Lombardo (Prov. of Bergamo) (IT); Ferrari, Adriano, 24069 Trescore Balneario (Prov. of Bergamo) (IT)
(74) Representative: Alagem Modiano, Lara S.

(56) References cited:
- WO-A-20/07081400
- US-A- 1 933 441
- US-A- 4 416 281
- US-A- 4 640 101
- US-A- 5 738 682
- US-A1- 2005 075 592

## Description

The present invention relates to a device for providing instant cooling, particularly for treating parts of the human body or items in general. More particularly, the invention relates to a device which is adapted to generate cold or heat instantly, so as to reduce or increase the temperature of parts of the human body for therapeutic purposes or to reduce or increase the temperature of items, such as helmets or bottle-carrying baskets.

As is known, for example after a specific surgical procedure, it is necessary to keep the affected part for a period of time at a temperature which is lower than the rest of the body in order to avoid the onset of acute inflammatory conditions. For this purpose, special plastic containers are normally used which contain chemical components which, when mixed together at the time of use, react and cause a drop in temperature. Such containers, placed in contact with the affected part, cool it.

Although these containers are widely used, their drawback is the fact that they have a preset duration, and once the chemical reaction has ended they remain inert, no longer generating cold, and in any case they do not offer temperature control. Substantially, this means that the containers go from being extremely cold when the chemical reaction is triggered, with discomfort for the person who uses them, and then gradually decrease their efficiency and become substantially useless.

Moreover, the entire surface of the containers reaches a low temperature, and this causes the moisture that is present in the environment to tend to condense on the containers, generating a copious production of water which is an additional discomfort for the person who uses such containers.

US 2005/075592 discloses a device as defined in the preamble of claim 1.

The aim of the present invention is to provide a device for providing instant cooling, particularly for application to body parts or items, which allows to lower the temperature of the body part or item when it is placed in contact with such part or item.

Within this aim, an object of the present invention is to provide a device for providing instant cooling which can be used at any time and whose duration is not limited by the duration of a chemical reaction which triggers cooling.

Another object of the present invention is to provide a device for providing instant cooling which can be carried conveniently in one of the user's pockets and can be applied in each instance to the body part to be treated.

Still another object of the present invention is to provide a device for providing instant cooling which is capable of maintaining a temperature which is always constant over time.

This aim and these and other objects, which will become better apparent hereinafter, are achieved by a device for providing instant cooling, particularly for treating parts of the human body or items in general, as defined in claim 1.

Further characteristics and advantages of the invention will become better apparent from the description of a preferred but not exclusive embodiment of the device according to the invention, illustrated by way of non-limiting example in the accompanying drawings, wherein:

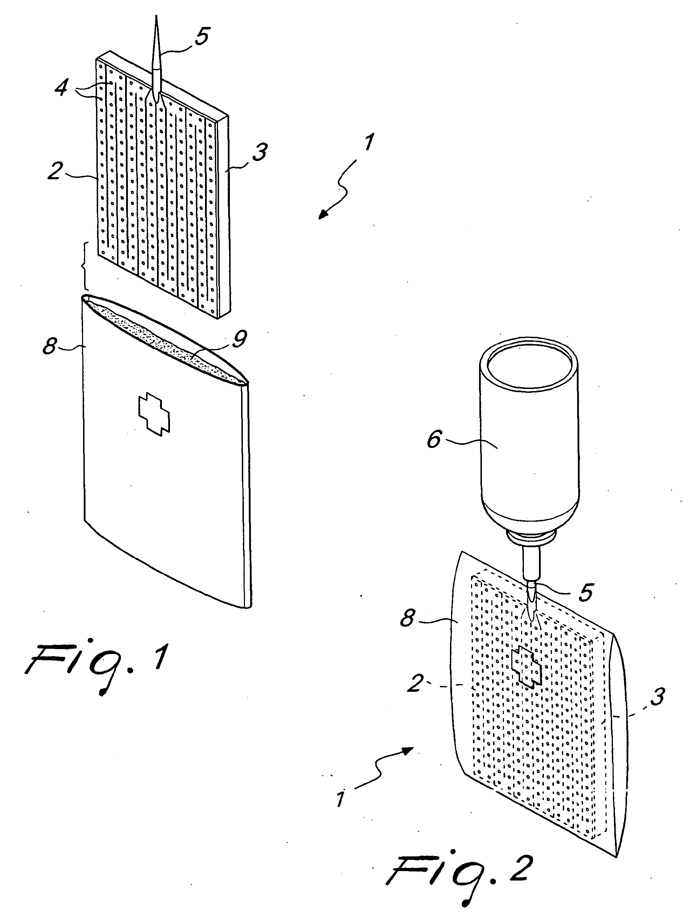

Figure 1 is an exploded perspective view of the device according to the present invention;
Figure 2 is a perspective view of the propellant bottle to be used in combination with the device according to the present invention;

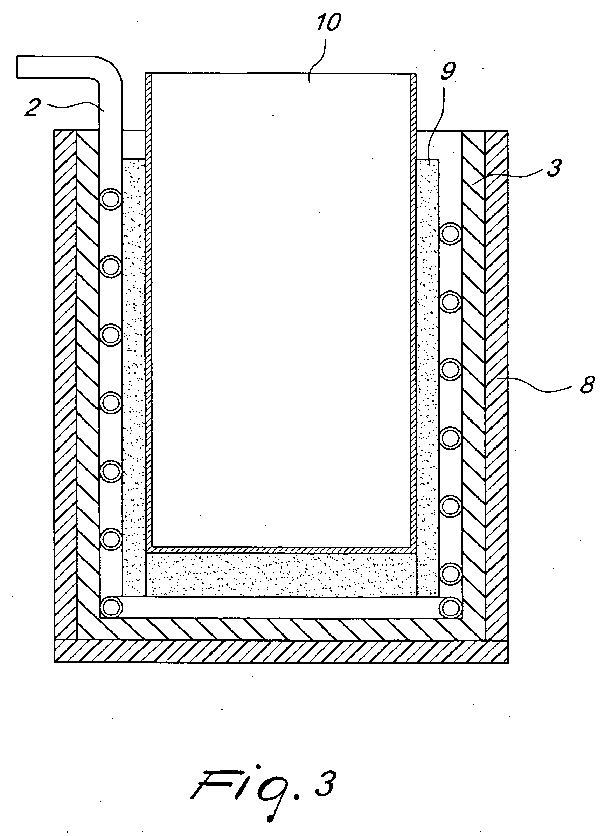

Figure 3 is a schematic view of the device according to the invention when used as a bottle-carrying basket.

With reference to the figures, the device according to the invention, generally designated by the reference numeral 1, comprises means for distributing a gas, which are constituted conveniently by a diffuser 2, which is made for example of sheet metal and is adapted to contain a gas which allows cooling. Conveniently, the diffuser 2 has, at its face designed to be gripped by the user's hand, a layer 3 of thermally insulating material so as to eliminate the discomfort that the user may experience when holding an extremely cold body.

The diffuser 2 has, at the surface that lies opposite the one on which the layer 3 of material is provided, a plurality of holes 4 which are adapted to allow the escape of the gas introduced in the diffuser 2.

Conveniently, the diffuser 2 is provided with means adapted to allow the introduction of the gas in the diffuser, provided for example by a nozzle 5 which allows to connect the diffuser 2 to a bottle 6 of gas, which when needed is connected to the nozzle 5 in order to inject gas into the diffuser 2, and therefore allows the escape of the gas through the holes 4.

Conveniently, the diffuser 2 is accommodated within a containment pouch or container 8, which contains a thermally insulating material, designated by the reference numeral 9, which is constituted for example by soapstone powder with a suitable particle size, which has the task of absorbing the low temperature generated by the expanding gas and of retaining it as long as possible as a function of the amount of soapstone powder and of the amount of gas.

The pouch 8 is sealed around the diffuser 2, with only the nozzle 5 protruding from the pouch 8, in order to allow the connection of the bottle 6 of propellant.

Operation of the device according to the present invention is as follows.

If it is necessary to have instant cold, the user connects the bottle 6 of propellant to the nozzle 5 of the diffuser 2, which is accommodated within the pouch 8, and the gas immediately begins to expand from the diffuser 2 through the holes 4 into the pouch 8, and the soapstone powder absorbs the low temperature that is thus generated.

The soapstone powder is designed to maintain as long as possible the selected low temperature, which can also be a function of the amount of gas injected into the diffuser 2. Therefore, depending on the application, it is possible to give the user an indication as to how much gas he will have to introduce in the diffuser 2 in order to obtain a given temperature.

The device according to the invention is perfectly reusable, since it is only necessary optionally to replace the bottle 6 of propellant in order to have a fresh fill of gas.

The device according to the invention is particularly compact in its dimensions, can be carried easily and can be applied at any time to any part of the body, or to any item, without the generated cold temperature undergoing rapid decay over time and most of all without the device, once in contact with the environment, forming condensation and generating a copious production of water, as occurs with known types of devices.

The diffuser 2 is made of very thin sheet metal, so as to allow it to be highly flexible, indeed to allow the user to adapt the diffuser 2, accommodated in the pouch 8, in the best possible manner to the affected body part.

The device according to the invention, with the same structure described above, can also be used for applications which are different from the one proposed above.

In particular, the device can be provided with an outer container or pouch 8 shaped like a basket and therefore can act as a bottle-carrying basket, so as to allow to preserve the bottles at a given temperature.

In this case, the container can be made of plexiglass or other material, with the layer 3 of thermally insulating material arranged internally to the container. The diffuser means 2 are then arranged, with the thermally insulating means 9 inside the container 8, leaving a free space at the center for accommodating a bottle-container 10.

Another possible application of the device according to the invention provides for its integration within a protective helmet, such as a motorcyclist's helmet and the like. In this case, the purpose would be to keep at a controlled temperature the head of the person who wears the helmet, preventing, especially in the summer months, the heat within the helmet from becoming excessive.

Further applications can provide for use for example in rucksacks, picnic bags and the like.

The device thus conceived is susceptible of numerous modifications and variations, all of which are within the scope of the appended claims; all the details may further be replaced with other technically equivalent elements.

In practice, the materials used, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device (1) for providing instant cooling, particularly for treating parts of the human body or items in general, comprising diffuser means (2) which are adapted to be charged with a gas, said diffuser means (2) being provided with a plurality of holes (4) for releasing said gas, said diffuser means (2) being accommodated in a container (8) which contains a thermally insulating material (9) which is adapted to absorb the low temperature generated by said gas, said container (8) being sealed around said diffuser means (2), **characterized in that** said diffuser means (2) are made of flexible sheet metal.

2. The device according to claim 1, **characterized in that** said diffuser means (2) are provided, at one of their surfaces, with a layer of thermally insulating material (9), which is arranged opposite the surface of said diffuser means (2) in which said plurality of holes (4) is defined.

3. The device according to one or more of the preceding claims, **characterized in that** said diffuser means (2)are provided with at least one nozzle (5) for injecting gas into said diffuser means (2)

4. The device according to one or more of the preceding claims, **characterized in that** said thermally insulating material (9) introduced in said container (8) is adapted to freeze upon contact with said gas.

5. The device according to one or more of the preceding claims, **characterized in that** said container is made of fabric.

6. The device according to one or more of the preceding claims, **characterized in that** said container (8) defines a central space to accommodate a bottle container, said bottle container being surrounded by said thermally insulating material (9) and said diffuser means (2).

7. A kit for providing instant cooling, particularly for treating body parts and items in general, **characterized in that** it comprises a device (1) according to one or more of the preceding claims and a bottle (6) of propellant which is adapted to inject gas into said diffuser means (2).

## Patentansprüche

1. Vorrichtung (1) für die Schaffung einer sofortigen Kühlung, insbesondere für die Behandlung von Teilen des menschlichen Körpers oder von Gegenständen im Allgemeinen, mit Diffusionsmitteln (2), die dazu ausgelegt sind, mit einem Gas beschickt zu werden, wobei die Diffusionsmittel (2) mit mehreren Löchern (4) versehen sind, um das Gas freizugeben, wobei die Diffusionsmittel (2) in einem Behälter (8) ausgenommen sind, der ein wärmeisolierendes Material (9) enthält, das dazu ausgelegt ist, die durch das Gas erzeugte tiefe Temperatur zu absorbieren, wobei der Behälter (8) um die Diffusionsmittel (2) abgedichtet ist, **dadurch gekennzeichnet, dass** die Diffusionsmittel (2) aus einem biegsamen Metallblech hergestellt sind.

2. Vorrichtung nach Anspruch 1, **dadurch gekennzeichnet, dass** die Diffusionsmittel (2) an einer ihrer Oberflächen mit einer Lage aus einem wärmeisolierenden Material (9) versehen sind, das gegenüber der Oberfläche der Diffusionsmittel (2), in der die mehreren Löcher (4) definiert sind, angeordnet ist.

3. Vorrichtung nach einem oder mehreren der vorhergehenden Ansprüche, **dadurch gekennzeichnet, dass** die Diffusionsmittel (2) mit wenigstens einer Düse (5) versehen sind, um Gas in die Diffusionsmittel (2) einzuleiten.

4. Vorrichtung nach einem oder mehreren der vorhergehenden Ansprüche, **dadurch gekennzeichnet, dass** das in den Behälter (8) eingeleitete wärmeisolierende Material (9) dazu ausgelegt ist, bei Kontakt mit dem Gas zu gefrieren.

5. Vorrichtung nach einem oder mehreren der vorhergehenden Ansprüche, **dadurch gekennzeichnet, dass** der Behälter aus Textilmaterial hergestellt ist.

6. Vorrichtung nach einem oder mehreren der vorhergehenden Ansprüche, **dadurch gekennzeichnet, dass** der Behälter (8) einen Mittelraum definiert, um einen Flaschenbehälter aufzunehmen, wobei der Flaschenbehälter von dem wärmeisolierenden Material (9) und von den Diffusionsmitteln (2) umgeben ist.

7. Bausatz für die Schaffung einer sofortigen Kühlung, insbesondere für die Behandlung von Körperteilen oder von Gegenständen im Allgemeinen, **dadurch gekennzeichnet, dass** er eine Vorrichtung (1) nach einem oder mehreren der vorhergehenden Ansprüche sowie eine Flasche (6) mit Treibmittel, das dazu ausgelegt ist, Gas in die Diffusionsmittel (2) einzuleiten, umfasst.

## Revendications

1. Dispositif (1) pour fournir un refroidissement instantané, en particulier pour traiter des parties du corps humain ou des éléments en général, comprenant des moyens de diffusion (2) qui sont adaptés pour être chargés d'un gaz, lesdits moyens de diffusion (2) étant munis d'une pluralité de trous (4) pour libérer ledit gaz, lesdits moyens de diffusion (2) étant contenus dans un récipient (8) contenant un matériau isolant thermique (9) adapté pour absorber la faible température générée par ledit gaz, ledit récipient (8) étant scellé autour des moyens de diffusion (2), **caractérisé par le fait que** lesdits moyens de diffusion (2) sont fabriqués dans de la tôle souple.

2. Dispositif selon la revendication (1), **caractérisé par le fait que** lesdits moyens de diffusion (2) sont dotés, au niveau de leur surface, d'une couche d'un matériau isolant thermique (9), disposée en face de la surface desdits moyens de diffusion (2) sur laquelle une pluralité de trous (4) est définie.

3. Dispositif selon une ou plusieurs des revendications précédentes, **caractérisé par le fait que** lesdits moyens de diffusion (2) sont équipés d'au moins une buse (5) pour injecter le gaz dans lesdits moyens de diffusion (2).

4. Dispositif selon une ou plusieurs des revendications précédentes, **caractérisé par le fait que** ledit matériau isolant thermique (9) introduit dans ledit récipient (8) est adapté pour geler au contact avec ledit gaz.

5. Dispositif selon une ou plusieurs des revendications précédentes, **caractérisé par le fait que** ledit récipient est en toile.

6. Dispositif selon une ou plusieurs des revendications précédentes, **caractérisé par le fait que** ledit récipient (8) définit un espace central pour recevoir une bouteille, ladite bouteille étant entourée dudit matériau isolant thermique (9) et par lesdits moyens de diffusion (2).

7. Kit pour fournir un refroidissement instantané, en particulier pour traiter des parties du corps humain et des éléments en général, **caractérisé par le fait qu'**il comprend un dispositif (1) selon une ou plusieurs des revendications précédentes et une bouteille (6) de propulseur qui est adaptée pour injecter le gaz dans lesdits moyens de diffusion (2).
